# EUROPEAN PATENT SPECIFICATION

(11) **EP 3 860 479 B1**
(45) Date of publication and mention of the grant of the patent: **05.06.2024**
(21) Application number: 19787454.8
(22) Date of filing: 03.10.2019
(51) Int. Cl.: A61B 17/34, A61B 90/00, A61B 17/32

(54) **INSERTION DEVICES**
EINSATZVORRICHTUNGEN
DISPOSITIFS D'INSERTION

(30) Priority: 03.10.2018 US 201862740506 P
(43) Date of publication of application: 11.08.2021
(73) Proprietor: Establishment Labs S.A., La Garita, Alajuela (CR)
(72) Inventor: SEIDNER H., Ariel, La Garita, Alajuela (CR); VIQUEZ, Jose Pablo, La Garita, Alajuela (CR); MELENDEZ, David, La Garita, Alajuela (CR); MORAN, Solange Vindas, La Garita, Alajuela (CR); RANDQUIST, Charles, 133 36 Saltsjöbaden (SE); DAJLES, Denise, La Garita, Alajuela (CR); DE MEZERVILLE, Roberto, La Garita, Alajuela (CR); VALERIO, Fernando, La Garita, Alajuela (CR); CHACÓN QUIRÓS, Juan José, La Garita, Alajuela (CR)
(74) Representative: Haseltine Lake Kempner LLP
(86) International application number: PCT/IB2019/058435
(87) International publication number: WO 2020/070693

(56) References cited:
- WO-A1-2011/140382
- US-A- 5 658 328
- US-A1- 2006 136 069
- US-A1- 2017 056 049
- US-B1- 6 540 764

## Description

### TECHNICAL FIELD

The present disclosure relates generally to devices and example methods for inserting expandable balloons into surgical sites.

### BACKGROUND

Breast implants are among the largest implantable medical devices used in the human body today. Due to their volume, mass, and surface area, these implants can cause unique physiological effects in the surrounding tissues. These effects may be caused in part by invasive techniques for introducing breast implants, which may in some cases cause trauma to the mammary tissue and surrounding tissues.

Current techniques for insertion of medical implants (e.g. breast implants) may create surgical wounds resulting in an extended, complex, and/or dynamic healing process, e.g., to allow a patient body to replace devitalized and missing cellular structures and/or tissue layers. For example, many current techniques require a relatively large incision at or near a surgical implantation site (e.g., a tissue pocket). The incision and/or implantation site may need to be manipulated by retractors and/or tissue-spreaders to expand and hold it open, while an implant is physically manipulated into the implantation site. Moreover, the incision and/or implantation site may need to be enlarged or expanded using retractors or tissue-spreaders, in order to accommodate an implant. Creation, expansion, and holding open of an implantation site may result in pain, scarring, infection at the implantation site, and/or other undesirable effects. In addition, larger incisions may increase the potential incidence of keloid and hypertrophic scarring, during and after healing. Certain patients are also more susceptible to, and are at higher risk of, keloid formation.

### SUMMARY

The invention is defined by claims 1 and 13. Further embodiments of the invention are defined by the dependent claims. Methods as such do not form part of the claimed invention.

The present disclosure includes insertion devices and related kits and example methods.

For example, the present disclosure includes an insertion device comprising a base having a longitudinal axis and defining a cavity; an expandable balloon disposed within the cavity in a collapsed configuration; and a flexible lumen extending proximally from the balloon to a pump, the flexible lumen fluidly connecting the balloon to the pump. The base may include a projection extending distally, parallel to the longitudinal axis, wherein at least a portion of the balloon is coupled to the projection. The balloon may be affixed to the projection with an adhesive and/or a longitudinal length of the projection may be at least half a longitudinal length of a tube that defines the cavity. According to some aspects, the device may include a cover coupled to the base and movable relative to the base to selectively cover and expose the cavity to deploy the balloon. The cover may be slidable relative to the base along the longitudinal axis, or wherein the cover is coupled to the base with a removable clip. The cover may be configured to completely enclose the balloon within the cavity. In any such insertion devices, a distal end of the base may include a tapered atraumatic tip, a proximal portion of the base may include a handle, and/or the base may include a plurality of measurement markings. Further, for example, the diameter of the balloon in the collapsed configuration is between about 10 mm and about 15 mm. The balloon may be in fluid communication with a supply of air. In at least one example, a cross-sectional dimension of the base is less than about 3 cm, such as from about 0.5 cm to 3 cm.

The present disclosure also includes kits that include an insertion device as described above and/or elsewhere herein. The kit may also include a cup defining an interior volume, the cup configured to be placed external to a target site and to limit expansion of the balloon inside the target site, optionally wherein the cup includes a side opening.

Aspects of the present disclosure are directed to an insertion device, including: a base having a longitudinal axis and defining a cavity, the base including a projection extending distally from a distal end portion of the cavity, parallel to the longitudinal axis; an expandable balloon coupled to the projection in a collapsed configuration; and a flexible lumen extending proximally from the balloon to a pump, the flexible lumen fluidly connecting the balloon to the pump.

Optionally, the balloon is affixed to the projection with an adhesive. Optionally, a longitudinal length of the projection is at least half a longitudinal length of a tube that defines the cavity. Optionally, a distal end of the base includes a tapered atraumatic tip. Optionally, the base comprises a rigid material. Optionally, a proximal portion of the base includes a handle. Optionally, the base includes a plurality of measurement markings. Optionally, a diameter of the balloon in the collapsed configuration is between about 10 mm and about 15 mm. Optionally, the balloon is in fluid communication with a supply of air. Optionally, a cross-sectional dimension of the base is less than about 3 cm, such as from about 0.5 cm to 3 cm.

Additionally, some aspects of the present disclosure are directed to a kit, comprising a device according to the present disclosure, and a cup defining an interior volume, the cup configured to be placed external to a target site and to limit expansion of the balloon inside the target site. Optionally, the cup includes a side opening.

Some aspects of the present disclosure are directed to an insertion device, comprising: a base including a proximal end portion, a distal end portion, and a cavity, the cavity including an opening; a balloon disposed within the cavity in a collapsed configuration; a cover movably coupled to the base, wherein the cover, when in a closed configuration with the base, restricts movement of the balloon; and a flexible lumen extending from the balloon through the proximal end portion of the base to a pump, the flexible lumen fluidly connecting the balloon to the pump.

Optionally, the distal end portion of the base is tapered. Optionally, the cover partially obstructs the opening of the cavity in the closed configuration with the base. Optionally, the cavity is disposed at or proximate the distal end portion of the base, and the device further comprises a handle at the proximal end portion of the base. Optionally, the cover is slidable relative to the base in a direction parallel to a proximal-distal axis of the base. Optionally, the cover is frangibly connected to the base. Optionally, the cover includes parallel extensions configured to restrict movement of the balloon.

Optionally, the base and cover define a length extending along a proximal-distal axis, and a width perpendicular to the length, wherein the length is greater than the width. Optionally, the base and the cover include complementary features that restrict rotational movement of the base and the cover relative to each other. Optionally, complementary features of the base and the cover permit the base and the cover to slide relative to each other along a proximal-distal axis. Optionally, the base and the cover are coupled to one another by a clip extending at least partly around a circumference of the device.

### BRIEF DESCRIPTION OF THE DRAWINGS

Embodiments of the present disclosure may be implemented in connection with aspects illustrated in the attached drawings. These drawings show different aspects of the present disclosure and, where appropriate, reference numerals illustrating like structures, components, materials, and/or elements in different figures are labeled similarly. It is understood that various combinations of the structures, components, and/or elements, other than those specifically shown, are contemplated and are within the scope of the present disclosure. Further, even if it is not specifically mentioned, aspects described with reference to one embodiment may also be applicable to, and may be used with, other embodiments.

Moreover, the present disclosure is neither limited to any single aspect or embodiment, nor to any combinations and/or permutations of such aspects and/or embodiments. Each aspect of the present disclosure (e.g., device, method, etc.) and/or variations thereof, may be employed alone or in combination with one or more of the other aspects of the present disclosure and/or variations thereof. For the sake of brevity, certain permutations and combinations are not discussed and/or illustrated separately herein. Notably, an embodiment or implementation described herein as "exemplary" is not to be construed as preferred or advantageous, for example, over other embodiments or implementations. Rather, it is intended to reflect or indicate the embodiment(s) is/are "example" embodiment(s).
FIGS. 1A-1F show views of an exemplary insertion device, according to some aspects of the present disclosure.
FIG. 2A illustrates another exemplary insertion device according to some aspects of the present disclosure.
FIG. 2B illustrates another exemplary insertion device according to some aspects of the present disclosure.
FIG. 3 is an illustration of an exemplary insertion device assembly including a balloon, according to aspects of the present disclosure.
FIGS. 4A-4D illustrate additional views of components of the exemplary insertion device of FIG. 3.
FIGS. 5A-5C illustrate views and components of another exemplary insertion device, according to aspects of the present disclosure.
FIGS. 6A and 6B illustrate views of a further exemplary insertion device, according to aspects of the present disclosure.
FIG. 7 illustrates an exemplary balloon and inflation tube, according to aspects of the present disclosure.
. FIG. 8 illustrates an exemplary balloon, inflation tube, and pump according to aspects of the present disclosure.
. FIG. 9 illustrates another exemplary balloon, inflation tube, and pump according to aspects of the present disclosure.
FIGS. 10A-10D illustrate views of another exemplary insertion device, its components, and accessories, according to aspects of the present disclosure.
FIG. 11 illustrates, in flow chart form, an exemplary method of using an insertion device, according to aspects of the present disclosure.

### DETAILED DESCRIPTION

Examples of the present disclosure relate to systems, devices, and example methods inserting an expandable component, such as a balloon, into an implantation site of a patient's body. Such systems, devices, and example methods may include an insertion component and an expandable component.

The terms and definitions provided herein control, if in conflict with terms and/or definitions of art. As used herein, the terms "comprises," "comprising," or other variations thereof, are intended to cover a non-exclusive inclusion such that a process, method, article, or apparatus that comprises a list of elements does not include only those elements, but may include other elements not expressly listed or inherent to such a process, method, article, or apparatus. Additionally, the term "exemplary" is used herein in the sense of "example," rather than "ideal." As used herein, the terms "about," "substantially," and "approximately," indicate a range of values within +/- 5% of a stated value.

The terms "proximal" and "distal" are used herein to refer to the relative and directional positions of the components of an exemplary introducer device. "Proximal" or "proximally" refers to a position relatively closer to an operator of a device. In contrast, "distal" or "distally" refers to a position relatively farther away from the operator of a device, and or closer to an interior of a patient body.

The present disclosure includes devices and example methods for accurately and precisely inserting expandable devices into the human body. While portions of the present disclosure refer to insertion of balloons, such as dissector balloons, into a patient's body (e.g., into the chest as part of, or in preparation for, breast implant surgery), the devices, systems, and example methods disclosed herein may be used for insertion of other devices or structures (e.g., tissue expanders, etc.) in other locations of the body (e.g., gluteal, calf, arm, back, hip, etc.). In some embodiments, devices, systems, and methods disclosed herein may provide for introduction of structures into a location of a human body (e.g., an implantation site) in minimally-invasive procedure.

Some aspects of the present disclosure provide a sterile, biocompatible insertion device for inserting an expandable device into the human body. Suitably, expandable devices according to the present disclosure may be partly or entirely flexible (e.g., elastomeric, collapsible, compressible, foldable, and/or resiliently deformable). In some embodiments, the expandable device may include a balloon comprising a sterile, biocompatible material. Expandable devices and/or insertion devices according to the present disclosure may have low-friction surface properties to facilitate smooth delivery and implantation of the implant within the body of the patient.

Insertion devices according to the present disclosure may be configured for insertion through relatively small incisions, such as an incision of about 4 cm or less, about 3 cm or less, about 2 cm or less, or about 1 cm or less, e.g., an incision from about 0.5 cm to about 4 cm, or from about 2 cm to about 3 cm. In some embodiments, for example, devices according to the present disclosure may be insertable through an incision of about 2 cm. While insertion devices according to the present disclosure may be insertable through incisions greater than, e.g., about 4 cm, it will be understood that some embodiments of the present disclosure may be of particular use during a minimally invasive procedure. Minimally invasive procedures may reduce damage to patient tissue at and around an implantation site/incision, thereby decreasing recovery time and reducing scarring at the insertion site(s).

In some embodiments, insertion devices according to the present disclosure may be sized to account for shapes and contours of an implantation site (e.g., contours of a breast area, gluteal area, calf area, etc.). In some embodiments, therefore, insertion devices according to the present disclosure may assist in the creation of a passage, opening, or canal between an incision and an implantation site, to promote safe and effective delivery of an expandable device to the desired site, with a suitable orientation.

Devices according to the present disclosure may be suitable for insertion through an incision in a patient's skin and introduction into a body cavity and/or between tissue planes or layers. In the case of an insertion device configured to deliver an expandable balloon to a chest area, for example, the insertion device may be advanced to the desired balloon deployment area, such as a space between major and minor pectoral muscles, below/posterior to the mammary gland(s). Once the insertion device is positioned in the desired area, the balloon may be deployed and inflated to assist with the creation and/or expansion of a cavity into which a breast implant is to be inserted, by, for example, dissecting tissue from other adjacent tissue. Additionally or alternately, such a balloon may be deployed to, e.g., stop or stanch bleeding generated during a tissue dissection process, by, e.g., applying direct pressure to an internal wound.

Insertion devices according to the present disclosure may have one unitary part, or may include multiple parts that may be assembled. A part of an insertion device configured for holding an expandable component, implant, or implement (e.g., a balloon, tissue expander, etc.) may be referred to herein as a base.

Reference will now be made to the figures of the present disclosure. It is to be understood that characteristics or aspects of one embodiment (e.g., sizes, shapes, attachments, materials, etc.) are applicable to each other embodiment disclosed herein. For brevity, characteristics shared amongst various embodiments will not be described repetitively herein.

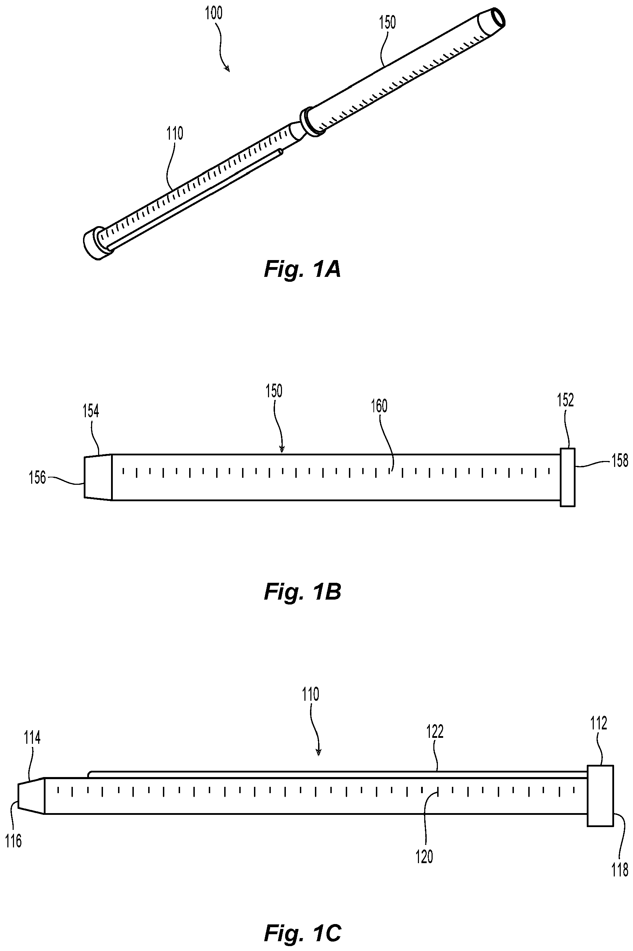

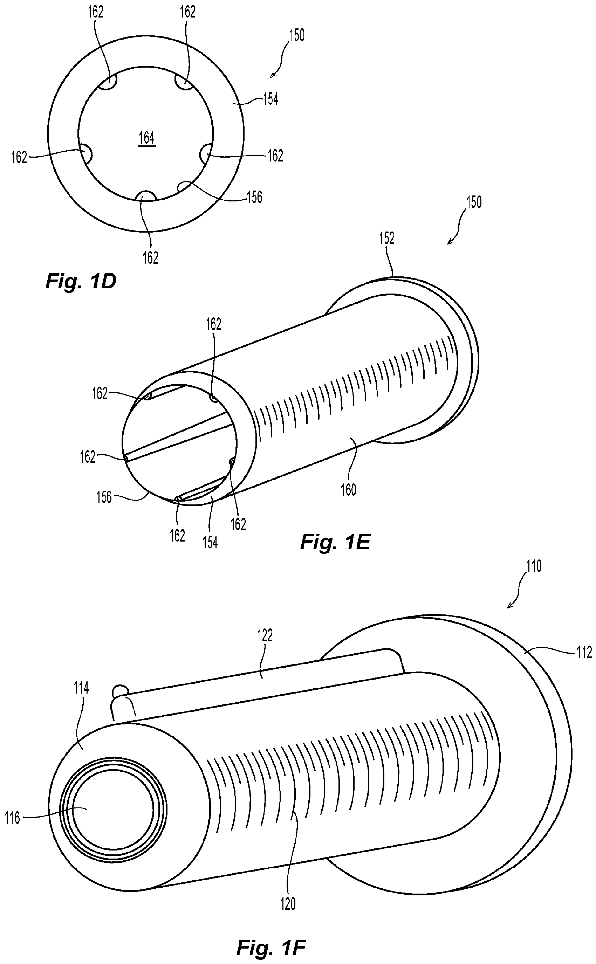

FIGS. 1A-1F show views of an exemplary insertion device 100. Insertion device 100 includes a base 150 and an inner member 110. Base 150 (see FIGS. 1A, 1B, 1D, 1E) may be generally cylindrical in shape, and may include a proximal end portion 152, a distal end portion 154, an open proximal end 158, and an open distal end 156, and may include depth markers 160. An interior 164 of base 150 (see, e.g., FIG. 1D) may include a plurality of interior protrusions 162 extending along a longitudinal length of base 150 (see, e.g., FIG. 1E), creating a plurality of longitudinal cavities or depressions between each protrusion. Inner member 110 (see FIGS. 1A, 1C, 1F) may include a proximal end cap 112 having a proximal side 118, a distal end portion 114, a distal tip 116, and a longitudinal ridge 122. Inner member 110 may also include depth markers 120.

Inner member 110 may be sized and configured to slide longitudinally into, and move axially with respect to, interior 164 of base 150. In some embodiments, inner member 110 and base 150 may be concentric cylinders or tubes, each defining a lumen therein. However, this example is non-limiting. For example, inner member 110 and base 150 may have any other shape allowing for inner member 110 to fit within (e.g., and slide relative to) base 150. In some embodiments, base 150 may define a lumen corresponding to interior 164, while inner member 110 may be solid or have a closed interior (that is, inner member 110 does not include a lumen).

The dimensions of inner member 110 may be selected to allow for inner member 110 to be disposed within, and slide relative to, base 150. In some embodiments, an outer diameter (or other cross-sectional dimension) of base 150 may range from about 1.0 cm to about 3.0 cm. For example, the outer diameter (or other cross-sectional dimension) of base 150 may range from about 1.5 cm to about 3.0 cm, from about 1.5 cm to about 2.5 cm, or from about 1.8 cm to about 2.0 cm, such as about 1.8 cm, 1.9 cm, or 2.0 cm. In some embodiments, an inner diameter (or other cross-sectional dimension) of base 150 may range from about 0.5 cm to about 2.8 cm, such as from about 0.5 cm to about 2.5 cm, from about 1.0 cm to about 2.0 cm, from about 1.5 cm to about 2.0 cm, or from about 1.6 cm to about 1.7 cm, such as about 1.5 cm, about 1.6 cm, about 1.7 cm, about 1.8 cm, or about 1.9 cm. Additionally or alternatively, an outer diameter (or other cross-sectional dimension) of inner member 110 may range from about 0.5 cm to about 2.5 cm, such as from about 0.5 cm to about 2.0 cm, from about 0.5 cm to about 1.5 cm, from about 1.0 cm to about 1.5 cm, or from about 1.2 cm to about 1.3 cm, such as about 1.0 cm, about 1.1 cm, about 1.2 cm, about 1.3 cm, about 1.4 cm, or about 1.5 cm.

In some embodiments, each of base 150 and inner member 110 has a length ranging from about 15 cm to about 35 cm, such as from about 18 cm to about 32 cm, from about 18 cm to about 30 cm, from about 18 cm to about 28 cm, from about 18 cm to about 25 cm, from about 19 cm to about 23 cm, or from about 19 cm to about 21 cm, such as about 16 cm, about 18 cm, about 20 cm, about 22 cm, about 24 cm, about 26 cm, about 28 cm, about 30 cm, about 32 cm, or about 34 cm. All of the above measurements are exemplary, and the measurements of embodiments of the present disclosure are not limited thereto. While the above measurements have been given with respect to insertion device 100, these measurements may also apply to dimensions of similar features of other exemplary insertion devices disclosed herein.

Base 150 may be configured to house an expandable device or component (e.g., a balloon, tissue expander, etc.) therein. Such an expandable device may be foldable or rollable in order to fit within interior 164 of base 150 in an undeployed configuration. Suitable expandable devices may include, e.g., a balloon, such as balloons 700, 800, 900 discussed further herein. Inner member 110, when inserted into interior 164 of base 150 at proximal end 158 of base 150, may be sized and configured to push an expandable device within base 150 distally, such that it is expelled from distal end 156 of base 150. In some embodiments, inner member 110 may also define a lumen, through which a tube may pass (e.g., a fluid supply tube) from a balloon or other expandable device within interior 164 of base 150 to a proximally-located pump or fluid source. Thus, a balloon or other expandable device to be inserted using insertion device 100 may be connected to, and in fluid communication with, a pump or fluid source in advance of being deployed in a surgical site.

As shown in FIGS. 1D and 1E, the inner wall of base 150 may include one or more interior protrusions 162, which may assist in reducing friction caused by relative movement of base 150 and inner member 110, and thus may assist in distal deployment of an expandable device from interior 164. Additionally, protrusions 162 may reduce or inhibit rotation of an expandable device about a longitudinal axis of base 150. Protrusions 162 may extend parallel to one another longitudinally along a length of base 150 (optionally along the entire length of base 150 or a portion thereof), and may protrude radially inward of the inner surface of base 150. While base 150 is depicted as including five protrusions 162, base 150 may alternately have one, two, three, four, six, seven, or more protrusions 162. In some embodiments, base 150 does not include protrusions 162.

Inner member 110 may include longitudinal ridge 122 that may cooperate with a corresponding space within base 150, e.g., a space in between two protrusions 162. Ridge 122 may be a longitudinally extension that protrudes radially outward from the outer surface of inner member 110, or the outer surface of inner member 110 may include ridge 122 as a integrated portion of inner member 110. According to some aspects herein the shape of ridge 122 may be chosen in order to fit between two protrusions 162 within base 150. This arrangement may assist in preventing or otherwise inhibiting rotation of inner member 110 relative to base 150, about a central longitudinal axis of the assembled insertion device 100. Additionally or alternatively, the outer surface of inner member 110 may include at least one or a plurality of protrusions (e.g., similar to protrusions 162) while the inner surface of base 150 may include one or more longitudinal ridges (e.g., similar to ridge 122).

Distal end portions 114, 154 of inner member 110 and base 150, respectively, may taper radially inward. This shape may assist with insertion of base 150 into an incision at an implantation site of interest, and may assist with insertion of inner member 110 into an open proximal end 158 of base 150. Proximal end portion 152 of base 150 and/or proximal end cap 112 of inner member 110 may each have larger diameters than a remainder of each of base 150 and inner member 110. This may allow for easier manipulation of insertion device 100 using proximal end portion 152 and proximal end cap 112, and may further serve as a stop during insertion of inner member 110 into interior 164 of base 150.

Base 150 and inner member 110 may include depth markers 160, 120 respectively. Depth markers 160, 120 may provide a visual aid to a user (e.g., a medical professional, such as a physician) during a medical procedure. During insertion of insertion device 100 into a body, for example, depth markers 160 may assist in proper positioning (e.g., achieving a proper insertion distance) of distal end portion 154 of base 150 in a desired location (e.g., a tissue pocket). Further, for example, depth markers 120 may assist in determining the distance inner member 110 has traveled into base 150 to determine whether inner member 110 has moved sufficiently to push an expandable device contained within interior 164 out of distal end 156 of base 150.

In some embodiments, insertion device 100 (that is, base 150 and/or inner member 110) may include a biocompatible lubricant, to reduce friction between the two components and promote moving more smoothly with respect to one another. Such a lubricant may also be used to slide an expandable device (e.g., a balloon) along interior 164 of base 150. The lubricant may be applied to an interior and/or an exterior of one or both of base 150 and inner member 110. The lubricant may be applied as a gel, a spray, or using any other suitable method. Additionally or alternately, inner member 110 and base 150 may be made of a material or have a coating that has a low friction coefficient, or otherwise produces a lubricious surface, allowing inner member 110, base 150, and/or an expandable device contained within base 150 to move smoothly with respect to each other.

According to some examples herein, distal end portion 154 of base 150 and/or distal end portion 114 of inner member 110 may include an LED or other light source, to aid in placement of insertion device 100 and/or an expandable device loaded within insertion device 100. The light emitted by the LED or other light source may be viewable through patient tissue and/or skin to ensure that distal end portion 154 and/or distal end portion 114 are positioned in the appropriate location(s) during use.

During an exemplary method of use, distal end portion 154 of base 150 may be placed into an incision in tissue. Base 150 may be pushed into a tissue pocket or other space (e.g., between tissue layers or planes). In some embodiments, base 150 may be pushed between two layers of tissue to dissect the layers from one another. Once distal end portion 154 is positioned at a desired area for deployment of an expandable device, the expandable device may be inserted into interior 164 of base 150 through open proximal end 158. Placement of an expandable device in interior 164 may include rolling or otherwise collapsing the expandable device upon itself to minimize its cross-sectional size. Once the expandable device is placed in interior 164, a flexible lumen (e.g., an inflation tube) connected to a proximal end of the expandable device may be inserted into a distal end 116 of inner member 110 and through inner member 110. A pump or fluid source (e.g., an inflation pump, a hand pump, pressurized fluid source, or other suitable device) may be attached to a proximal end of the flexible lumen (opposite the end attached to the expandable device within interior 164 of base 150).

In some embodiments, placement of an expandable device into interior 164 and placement of the flexible lumen through inner member 110 may be accomplished prior to inserting base 150 into an incision.

Once the expandable device is positioned in base 150 and the flexible lumen is exposed from a proximal end of inner member 110, a distal end portion 114 of inner member 110 may be slid into proximal end 158 of base 150. As inner member 110 is advanced distally towards distal end portion 154 of base 150, distal end portion 114 of inner member 110 may push the expandable device distally along interior 164 of base 150. As distal end portion 114 nears distal end portion 154, the expandable device is pushed distally out of open distal end 156, into a desired position (e.g., between tissue layers or planes to be dissected or in an otherwise desirable location).

Once the expandable device is inserted, a user may expand the expandable device by supplying fluid to the expandable device. The fluid may comprise, for example, a gas (e.g., an inert or relatively inert gas such as air, nitrogen, helium, etc.) or a liquid (e.g., water, saline solution, etc.). Expanding the expandable device may achieve various results, such as expanding a tissue pocket or other cavity within a body, dissecting tissue, and/or applying pressure to internal tissue to, e.g., stop or reduce bleeding during a surgery. Base 150 and inner member 110 may remain disposed in the patient tissue during this time, or may be removed. To remove base 150 and inner member 110, any pump or fluid supply attached to a proximal end of a flexible lumen may be detached, such that base 150 and inner member 110 may be slid off of the proximal end of the lumen. After use of the expandable device is complete, it may be collapsed (e.g., deflated, contracted, or otherwise collapsed) and retracted into interior 164 via open distal end 156 by pulling a proximal end of the flexible lumen attached to the expandable device. Base 150, inner member 110, and the expandable device disposed within base 150 may all be removed from the incision.

After removing the expandable device and insertion device 100, a medical procedure may continue or be completed (including, e.g., insertion of an implant, suturing of an incision, etc.).

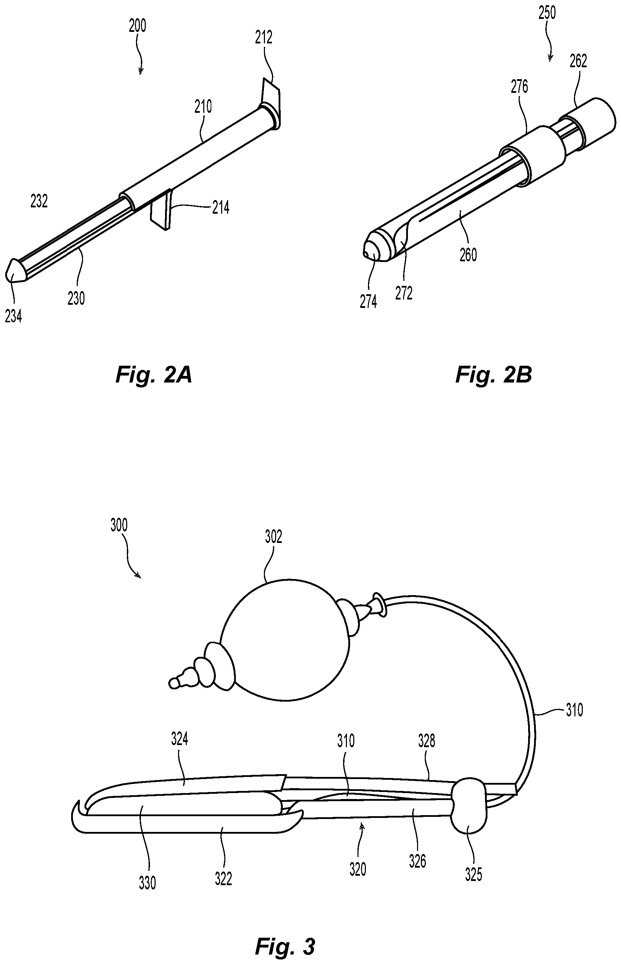

FIG. 2A illustrates another exemplary insertion device 200 according to some aspects of the present disclosure. Insertion device 200 as shown includes base 230 and cover 210, which may be movable (e.g., slidable) with respect to base 230. While insertion device 200 is depicted as having a generally cylindrical shape, it may have any shape suitable to insert an expandable device into an implantation site, a site for dissection, or other surgical site.

Base 230 and cover 210 may define a cavity (e.g., cavity 232), wherein base 230 and cover 210 may slide axially relative to one another to expose or close cavity 232. An expandable device such as a balloon may be placed in cavity 232 in a compact configuration (e.g., a rolled or otherwise collapsed configuration meant to reduce or minimize the cross-sectional dimension of the expandable device) when cavity 232 is exposed (that is, not closed by cover 210). Cover 210 may then be moved with respect to base 230 (e.g., axially slid distally relative to base 230) to cover and close cavity 232, and to restrict removal of the expandable device. Base 230 and cover 210 may include an open proximally-facing end or aperture through which a flexible lumen may extend to provide, e.g., a fluid supply to the expandable device within insertion device 200. As insertion device 200 is inserted into an incision, the flexible lumen may remain outside of the incision to allow for expansion of the expandable device, by introduction of fluid into the expandable device when the expandable device is deployed.

Base 230 may include one or more handles 212, 214, which may serve as grips for a user using insertion device 200 (e.g., to move cover 210 relative to base 230, or to move insertion device 200 as a whole in a proximal or distal direction). Additionally or alternately, such handles may serve as stops or obstructions, e.g., to prevent over-insertion of insertion device 200 into an incision, or a circumferential clip, closure, or ring preventing radial separation of cover 210 and base 230. Handles 212, 214 may be any size or shape to allow a user to manipulate insertion device 200 and/or to serve as obstructions. While two handles (212, 214) are depicted, in some embodiments, insertion device 200 may only include one handle. Additionally or alternately, insertion device 200 may include one or two of grips 262, 276 depicted in FIG. 2B, either in combination or instead of one or both handles 212 214. The distal end portion 234 of insertion device 200 may be tapered to, e.g., allow for ease of insertion of insertion device 200 into an incision and/or a site of interest. Further, an LED or other light source may be disposed at distal end portion 234, similar to insertion device 100, to assist in the placement and delivery insertion device 200 and/or an expandable device inside it.

One or more longitudinal ridges and/or interior protrusions (e.g., similar to longitudinal ridge 122 and interior protrusions 162 of insertion device 100) may be provided on either or both of base 230 and/or cover 210, to allow base 230 and cover 210 to interact and slide longitudinally with respect to one another without axially rotating relative to one another. A lubricant may be applied to reduce friction between base 230, cover 210, and or an expandable device in cavity 232. Additionally or alternately, one or more parts of insertion device 200 may be made using a lubricious material or coating.

FIG. 2B illustrates a further exemplary insertion device 250 according to aspects of the present disclosure, depicted at an approximately 90-degree angle of rotation relative to insertion device 200 depicted in FIG. 2A. Insertion device 250 may include base 270 and cover 260, which may be movable (e.g., slidable) with respect to base 270. While insertion device 250 is depicted as having a generally cylindrical shape, it may have any shape suitable to insert an expandable device into an implantation site, a site requiring dissection, or other surgical site.

Base 270 and cover 260 may define a cavity (e.g., cavity 272), wherein base 270 and cover 260 may slide axially relative to one another to expose or close cavity 272. An expandable device, when in a compact configuration (e.g., a rolled or otherwise collapsed configuration meant to reduce or minimize its cross-sectional dimension) may be placed in cavity 262 when cavity 272 is not closed by cover 260. Cover 260 may then be moved with respect to base 270 (e.g., axially slid distally relative to base 270) to cover and close cavity 272, and to restrict removal of the expandable device. Similar to cover 210 of insertion device 200, cover 260 may include an open proximally-facing end, through which a flexible lumen may extend to provide, e.g., a fluid supply to the expandable device within insertion device 250. As insertion device 250 is inserted into an incision, the flexible lumen remains outside of the incision to allow for expansion of the expandable device, by introduction of fluid into the expandable device when the expandable device is deployed.

Base 270 may include grips 276, 262, which a user may grip while using insertion device 250 (e.g., to move cover 260 relative to base 270, or to move insertion device 250 as a whole in a proximal or distal direction. Additionally or alternatively, grip 276 may serve as a circumferential clip, closure, or ring preventing radial separation of cover 260 and base 270. Additionally or alternately, grips 276, 262 may be obstructions to prevent over-insertion of insertion device 250 into an incision. In some embodiments, insertion device 250 may include only one or the other of grips 276, 262. Additionally or alternately, insertion device 250 may include one or two of grips 212, 214 depicted in FIG. 2A, either in combination or instead of one or both grips 276, 262. A distal end portion 274 of insertion device 200 may be tapered to, e.g., allow for ease of insertion of insertion device 200 into an incision and/or a site of interest. Further, an LED or other light emitting device may be disposed at distal end portion 274, to assist in the placement and delivery insertion device 250 and/or an expandable device inside it.

In an exemplary method of use of insertion device 200 or insertion device 250, the distal end portion (e.g., 234 or 274) may be placed into an incision near a site of interest, and the insertion device (200 or 250) may be inserted into the site of interest (e.g., into a tissue pocket or other cavity, between tissue layers or planes for dissection from one another, or otherwise into a site of interest). Once the distal end portion (e.g., 234 or 274) is positioned at a desired location for deployment of an expandable device from the cavity of the insertion device (e.g., cavity 232 or cavity 272), a user may slide the cover (e.g., cover 210 or cover 270) proximally to expose the expandable device disposed in the cavity (e.g., cavity 232 or cavity 272).

As with the insertion device 100 in FIGS. 1A-1F, the insertion device (e.g., insertion device 200 or 250) may remain in the incision while the expandable device is deployed. To deploy the expandable device, a user may attach a pump, fluid source, or other expansion mechanism to a proximal end portion of a flexible lumen attached to the expandable device, and may use the expansion mechanism to expand the expandable device. Expansion of the expandable device causes the expandable device to become too large for the cavity in which it is disposed (e.g., cavity 232 or cavity 272), thus resulting in the expandable device being deployed from the cavity. Further expansion of the expandable device may be performed to achieve a desired result (e.g., dissection of tissue layers or planes, application of pressure to an internal wound, expansion of an internal tissue pocket or other cavity, or other surgical result).

Alternatively, the insertion device (e.g., insertion device 200 or 250) may be removed from an incision prior to expanding the expandable device. As discussed with respect to insertion device 100, the insertion device (e.g., 200 or 250) may be slid proximally over the flexible lumen and removed prior to attachment of a pump, fluid supply, or other mechanism to a proximal end portion of the flexible lumen. Once the insertion device is outside of the incision, and the flexible lumen attached to the expandable device is removed from the proximal opening of the insertion device, a pump, fluid supply, or other expansion mechanism may be attached to the flexible lumen, and a user may expand the expandable device. After the desired use of the expandable device is complete (e.g., dissecting tissue, applying pressure to blood flow, or other use), the expandable device may be contracted or deflated, and may be removed from the target site via the incision. In the event the insertion device is removed prior to expanding the expandable device, the contracted expandable device may be removed through a canal created by the insertion device.

If the insertion device (e.g., insertion device 200 or 250) is not removed prior to expanding the expandable device, then the expandable device may be deflated and removed with the insertion device by moving both proximally, until both the insertion device and the expandable device are outside the patient. In some instances, the physician may first pull the flexible lumen connected to the expandable device in a proximal direction, so the expandable device is retracted into the cavity (e.g., cavity 232 or 272) of the insertion device. Once the expandable device is thus retracted, the cover of the insertion device (e.g., cover 210, 260) may be moved distally to restrict removal of the expandable device from the cavity. The insertion device (e.g., insertion device 200 or 250), containing the expandable device within the cavity (e.g., cavity 232 or 272), may be pulled in a proximal direction and removed from the incision.

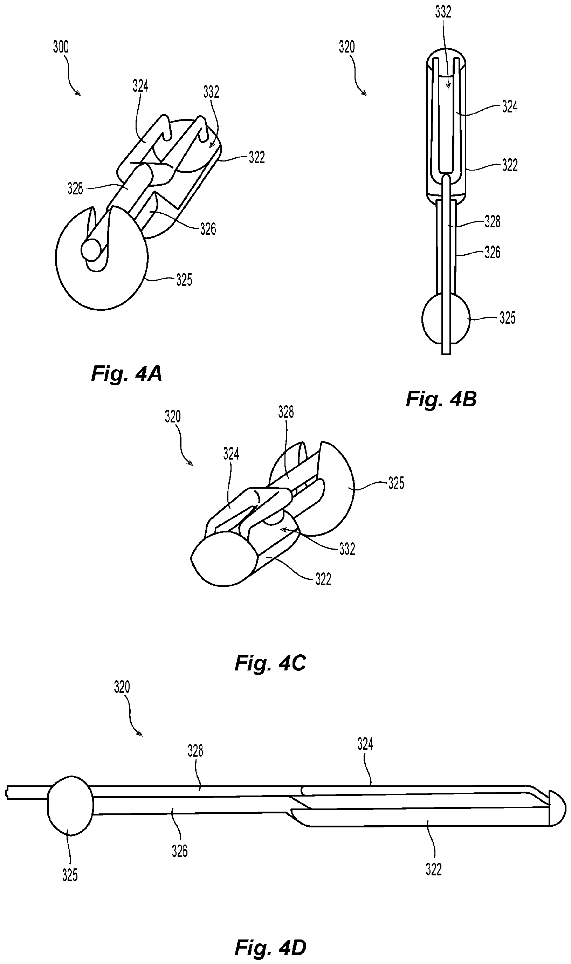

FIG. 3 is an illustration of an exemplary insertion device assembly 300, according to aspects of the present disclosure, with FIGS. 4A-4D showing additional views of components of the assembly 300. Insertion device assembly 300 includes an insertion device 320, which includes base 322 and cover 324 that define a cavity 332 (see FIG. 4B). Base 322 includes a support 326, and cover 324 includes a rod 328. Support 326 is connected to a handle 325, which includes a groove through which rod 328 may pass. Insertion device assembly 300 further includes a pump 302, a flexible lumen 310, and a balloon 330 (expandable device) in a folded configuration, disposed with in a cavity 332 of insertion device 320 (see, e.g., FIGS. 4A-4D). FIGS. 4A-4D illustrate additional views of insertion device 320 (insertion device assembly 300 without pump 302, lumen 310, or balloon 330).

Cavity 332 of insertion device 300 may be sized and configured to house balloon 330 in a folded state. Cover 324 may secure balloon 330 within cavity 332 during insertion of device 300 into an incision and a target site, and may be coupled, attached, or otherwise affixed to base 322 by a tab, a clip connection, or any other suitable coupling mechanism. Cover 324 may include parallel extensions (prongs), e.g., in a generally forkshaped configuration as shown, or may have other shapes or configurations (e.g., a plurality of bars, rods, or other blocking shapes) configured to restrict movement of balloon 330. In the example shown, the prongs of cover 324 extend axially over cavity 332 to secure balloon 330 within cavity 332. Distal ends of the prongs may be configured to protrude into cavity 332, to better secure balloon 330 within cavity 332, and/or to avoid catching or snagging patient tissue as insertion device 320 is inserted. Distal ends of the prongs optionally may include projections or protrusions configured to interact with corresponding features on base 322 (e.g., a notched portion or portions), to lock or otherwise secure cover 324 to base 322.

Handle 325 may have any suitable configuration or shape that allows a user to grip insertion device 320. For example, handle 325 may be generally spherical, as shown, or may have any other suitable shape (e.g., ergonomic, rectangular, etc.). Rod 328 of cover 324 may snap-fit or friction-fit into a groove in handle 325. Alternatively and/or additionally, rod 328 may rest in a groove in handle 325. During use, a user may grasp insertion device 320 such that rod 328 is secured in the groove of handle 325. Any other mechanism, such as a clip, a tab, or an adhesive, may be used to secure rod 328 to handle 325 or otherwise to secure cover 324 to base 322. In some embodiments, for example, cover 324 may be frangibly connected to base 322.

As shown, balloon 330 (or any other expandable device used in conjunction with insertion device 320) may be partly exposed to tissue at, e.g., a target site. For example, cover 324 as shown in FIGS. 4A-4C does not fully envelop balloon 330 and/or cavity 332. Alternatively, cover 324 may have a shape and size configured to completely enclose balloon 330 within cavity 332.

Materials, sizes, shapes, characteristics, measurements of parts of insertion device 320 and insertion device assembly 300 (e.g., dimensions, materials, etc. of insertion device 320 and insertion device assembly 300) may correspond to like materials, sizes, shapes, characteristics, and measurements of any other embodiment(s) disclosed herein. For example, insertion device 320 may include a rigid biocompatible material such as silicone, capable of sustaining forces applied thereto during an insertion procedure. Any suitable biocompatible lubricant or lubricious coating may be applied to part or all of insertion device assembly 300, e.g., to assist in moving cover 324 with respect to base 322, moving base 322 and cover 324 with respect to patient tissue, and/or moving balloon 330 and/or lumen 310 with respect to base 322, cover 324, and/or patient tissue. As with other exemplary insertion devices disclosed herein, a light source such as an LED or other visual indicator may be disposed at or proximate the distal end of insertion device 320, e.g., to assist in positioning the device 320. As with other exemplary devices disclosed herein, insertion device 320 may have a suitable longitudinal length (extending along a proximal-distal axis) for performing an insertion or implantation procedure. For example, the insertion device 320 may have a longitudinal length ranging from about 15 cm to about 35 cm, such as from about 18 cm to about 32 cm, from about 18 cm to about 30 cm, from about 18 cm to about 28 cm, from about 18 cm to about 25 cm, from about 19 cm to about 23 cm, or from about 19 cm to about 21 cm, such as about 16 cm, about 18 cm, about 20 cm, about 22 cm, about 24 cm, about 26 cm, about 28 cm, about 30 cm, about 32 cm, or about 34 cm. As is the case with other devices disclosed herein, the cross-sectional dimensions (perpendicular to a longitudinal length measured along a proximal-distal axis) of insertion device 500 may be relatively small (e.g., less than about 3 cm, less than about 2 cm, or less than about 1 cm across) to promote minimally-invasive procedures and minimize scarring or other tissue trauma.

Balloon 330, lumen 310, and pump 302 are exemplary, and may be used in any other exemplary device or assembly disclosed herein. Further, these components may share characteristics with any other embodiment disclosed herein, such as, e.g., expandable devices 700, 800, 900.

In an exemplary method of use, a distal end of insertion device assembly 300 (that is, distal end of insertion device 320) may be placed into an incision, and may be maneuvered to a target site (e.g., between tissues for dissection, to an internal tissue pocket or other cavity, to an area for application of pressure, etc.). A proximal end of lumen 310, along with pump 302, may remain outside the incision (i.e., outside the patient).

Once cavity 332 of insertion device 320 is positioned at a desired area, balloon 330 may be deployed by exposing cavity 332. To do so, cover 324 may be moved relative to base 322, and optionally removed from base 322. A user may inflate balloon 330 using pump 302 (this introducing a fluid such as air or other gas, or a liquid), while cover 324 and base 322 remain disposed at or near the target site. Upon completion of the procedure, balloon 330 may be collapsed and retracted back into cavity 332 by, e.g., pulling a proximal end of lumen 310 external to the incision. Insertion device 320 may then be removed from the patient.

Alternatively, once balloon 330 is deployed but before it is inflated, cover 324 and base 322 may be removed from the target site. For example, cover 324 and base 322 may be axially moved in the proximal direction until they pass distally through the incision. The pump may be attached to a proximal end of lumen 310 before or after cover 324 and base 322 have been removed. Balloon 330 may then be inflated. After inflation is completed as desired, balloon 330 may be collapsed and retracted by, e.g., pulling on a proximal end of lumen 310. After removing balloon 330, a user may complete a medical procedure, such as inserting a breast implant into a tissue pocket or cavity formed by the inflation of balloon 330.

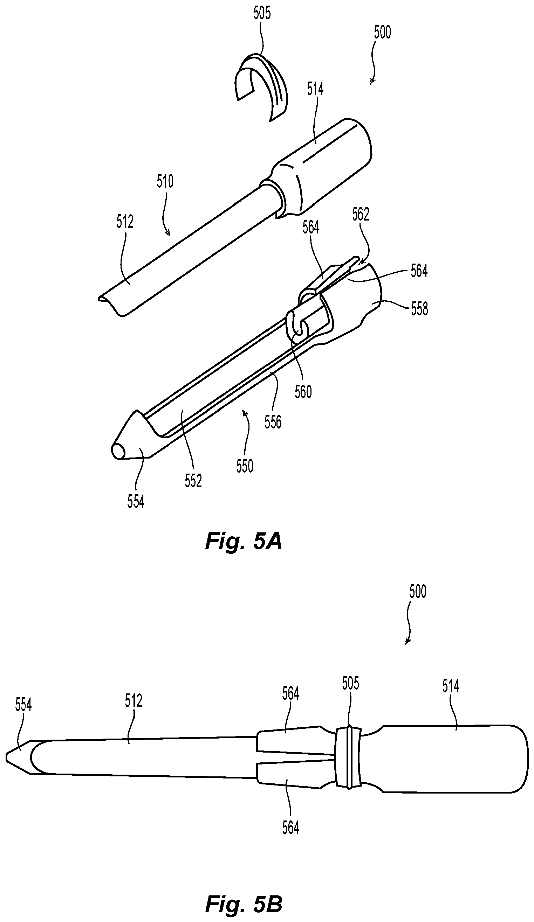

FIGS. 5A-5C illustrate views of another exemplary insertion device 500 according to aspects of the present disclosure. Insertion device 500 includes base 550, cover 510, and clip 505. Base 550 includes a body 556 defining a cavity 552, a distal end portion 554, and a handle portion 558 defining a groove 560. Handle portion 558 includes two curved wings 564, separated by a slot 562. Cover 510 includes a lid 512 complementary to cavity 552, and a handle portion 514.

Cover 510 may be coupled to, e.g., removably fixed to, base 550 by way of clip 505, which may have an opening configured to receive cover 510 and at least part of base 550. Clip 505 may have an opening configured to receive base 550 and at least part of cover 510. Clip 505 may include one or more mating features that cooperate with complementary mating features of base 550 and/or cover 510 (e.g., a groove, a detent, a snap fit connection, etc.).

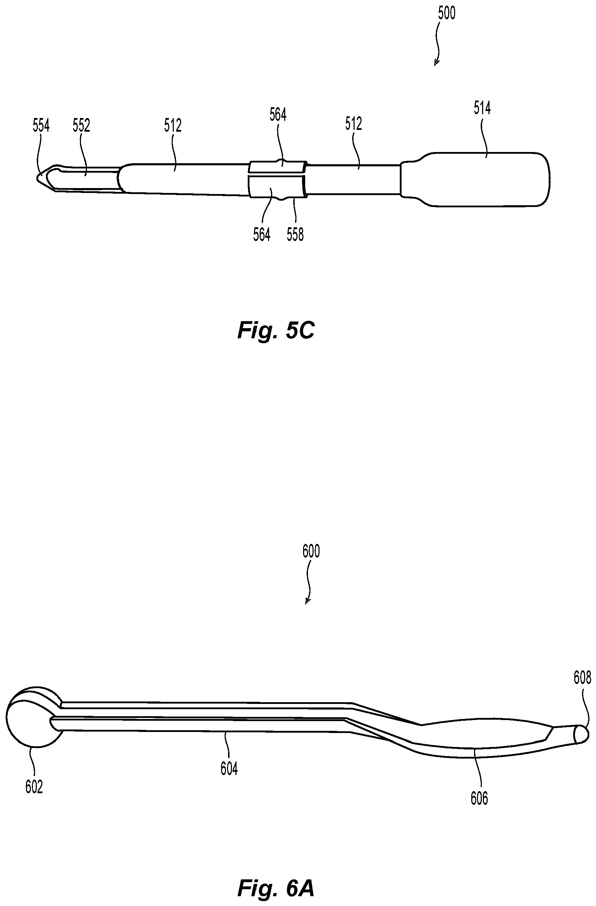

Handle portion 514 of cover 510 may have a concave shape configured to fit over a portion of base 550, such as groove 560. Handle portion 514 optionally may be configured to fit within (e.g., radially inward of) handle portion 558, for example, by passing between wings 564, through slot 562. Clip 505 may secure cover 510 to base 550 after handle portion 514 and handle portion 558 have been joined. In this manner, cover 510 may be fastened to base 550 (shown in, e.g., FIG. 5B). Clip 505 may be located distally from both handle portion 514 and handle portion 558, or may be positioned anywhere else suitable for securing cover 510 to base 550. When clip 505 is disengaged, cover 510 may be moved axially in a proximal direction relative to base 522, subject to pressure placed on wings 564 (e.g., radially inward) (shown in, e.g., FIG. 5C). In this manner, the axial movement of cover 510 may be controlled by, e.g., removal of clip 505 and by pressure on wings 564. Cover 510 may be moved proximally until an entirety of lid 512 is proximal to cavity 552 of base 550.

In some aspects, cover 510 may also be removed by pulling it in a substantially perpendicular direction to the axial extension of base 550. This movement may allow wings 564 to bend outward, away from a central axis of insertion device 500, allowing handle portion 514 to move through slot 562 and disengaging cover 524 from base 550. Cover 524 may then be fully removed.

Materials, sizes, shapes, characteristics, measurements of parts of insertion device 500 (e.g., dimensions, materials, etc. of insertion device 500) may correspond to like materials, sizes, shapes, characteristics, and measurements of any other examples disclosed herein. For example, insertion device 500 may include a rigid biocompatible material, such as silicone, e.g., capable of sustaining forces applied thereto during an insertion procedure. Wings 564 of insertion device 500 may include a semi-rigid material, such as, e.g., a semi-rigid polymer, allowing wings 564 to flex in order to accommodate their movement as described herein.

Any suitable biocompatible lubricant or lubricious coating may be applied to insertion device 500 to assist in moving parts of insertion device 500 relative to other parts, and/or moving insertion device 500 relative to tissue. An LED, other light source or other visual indicator may be provided at the distal end of insertion device 500 to assist in its positioning. As with other examples disclosed herein, insertion device 500 may have a longitudinal length (extending along a proximal-distal axis) ranging from about 15 cm to about 35 cm, such as from about 18 cm to about 32 cm, from about 18 cm to about 30 cm, from about 18 cm to about 28 cm, from about 18 cm to about 25 cm, from about 19 cm to about 23 cm, or from about 19 cm to about 21 cm, such as about 16 cm, about 18 cm, about 20 cm, about 22 cm, about 24 cm, about 26 cm, about 28 cm, about 30 cm, about 32 cm, or about 34 cm. As is the case with other devices disclosed herein, the cross-sectional dimensions (perpendicular to a longitudinal length measured along a proximal-distal axis) of insertion device 500 may be relatively small (e.g., less than about 3 cm, less than about 2 cm, or less than about 1 cm across), e.g., suitable for minimally-invasive medical procedures.

In an exemplary method of use, a distal end of assembled insertion device 500 (e.g., distal end portion 554) may be inserted into an incision and towards a target site. An expandable device may be provided in a collapsed configuration inside cavity 552, covered by lid 512. A flexible lumen, suitable for providing air or other fluid to the expandable device, may be disposed through a proximal end of device 500, through, e.g., groove 560 and into attachment with the expandable device in cavity 552. Alternately, a flexible lumen may extend proximally from cavity 552 without passing through a proximal end of device 500.

Once cavity 552 is positioned at a target site, the expandable device may be deployed. To do so, clip 505 may be removed, and cover 510 may be slid proximally relative to base 550 (alternately, cover 510 may be moved perpendicular to the central longitudinal axis of base 550, twisting it until it is pulled free from wings 564 through slot 562). Removing cover 510 exposes the expandable device within cavity 552. The expandable device may then be expanded, while base 550 and cover 510 remain within the incision. Following the expansion procedure, the expandable device may be collapsed (e.g., by extracting fluid from the expandable device) and retracted into cavity 552 (e.g., by pulling on a fluid supply lumen attached to the expandable device). Base 550, cover 510, and the expandable device within cavity 552 may then be retrieved through the incision.

Alternatively, prior to expanding the expandable device, cover 510 and base 550 may be removed from the target site (e.g., by axially moving them in the proximal direction, through the incision). A pump may be attached to a proximal end of the lumen connected to the expandable device either before or after cover 510 and base 550 are removed. The expandable device may then be expanded. Following expansion, the expandable device may be collapsed and pulled from the target site using the lumen connected to the expandable device. A medical procedure may then be continued or completed as needed (e.g., an implant may be inserted into the expanded area created by the expandable device).

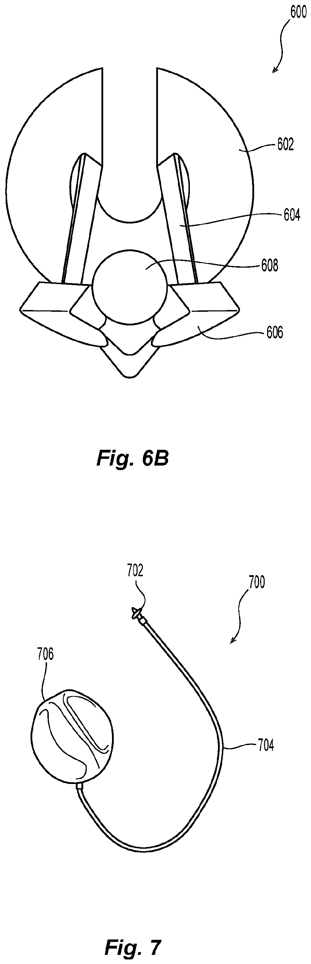

FIGS. 6A and 6B illustrate views of a further exemplary insertion device 600, according to aspects of the present disclosure. Insertion device 600 includes handle 602, body 604 (which may serve as a base), spoon-shaped recess 606, and distal tip 608. FIG. 6A depicts a side view of insertion device 600, while FIG. 6B depicts a view of the distal end of device 600.

Materials, sizes, shapes, characteristics, measurements of parts of insertion device 600 (e.g., dimensions, materials, etc. of insertion device 600) may correspond to like materials, sizes, shapes, characteristics, and measurements of any other examples disclosed herein. For example, insertion device 600 may include a rigid biocompatible material, such as silicone, capable of sustaining forces applied thereto during an insertion procedure. A distal end portion (e.g., distal tip 608) of insertion device 600 may include an LED or other light source or visual indicator to aid in accurate insertion and positioning of an expandable device.

As with other examples disclosed herein, insertion device 600 may have a longitudinal length (extending along a proximal-distal axis) ranging from about 15 cm to about 35 cm, such as from about 18 cm to about 32 cm, from about 18 cm to about 30 cm, from about 18 cm to about 28 cm, from about 18 cm to about 25 cm, from about 19 cm to about 23 cm, or from about 19 cm to about 21 cm, such as about 16 cm, about 18 cm, about 20 cm, about 22 cm, about 24 cm, about 26 cm, about 28 cm, about 30 cm, about 32 cm, or about 34 cm. As is the case with other devices disclosed herein, the cross-sectional dimensions (perpendicular to a longitudinal length measured along a proximal-distal axis) of insertion device 600 may be relatively small (e.g., less than about 3 cm, less than about 2 cm, or less than about 1 cm across) for use in minimally-invasive medical procedures. Moreover, an expandable device for use with insertion device 600 may adapt to the shape of a surrounding environment, such that insertion device 600 may maneuver the expandable device to a desired position at a target site via a relatively small incision, causing minimal trauma to tissue. As has been described elsewhere herein, a lubricant or lubricious coating optionally may be included on part or all of insertion device 600, to aid in moving insertion device 600 and/or an expandable device thereon through patient tissue.

Handle 602 may include a generally spherical component having a groove therein. The sphere-like shape may improve manipulability of insertion device 600, while the groove may allow for passage of a lumen from, e.g., a pump and/or fluid supply to an expandable device disposed in recess 606. It will be understood that handle 602 can be any shape suitable for allowing a user to manipulate insertion device 600. For example, in some embodiments, handle 602 may be ergonomic, rectangular, or any other shape that aids a user in maneuvering insertion device 600.

Body 604 may include a groove, into which a flexible lumen of an expandable device (e.g., an inflation tube for a balloon) may be positioned, such that the lumen may extend distally beyond handle 602, along a length of body 604, to recess 606 where it may be in fluid communication with an expandable device. The groove may extend along a full longitudinal length of body 604, as well as through handle 602. The groove may create or maintain a reduced profile for insertion device 600, which may be beneficial in preventing trauma, snagging of the lumen, or other complications while inserting device 600 into an incision during a medical procedure.

Like other examples disclosed herein, the distal tip 608 of insertion device 600 may be tapered and/or rounded (having rounded edges), to allow for ease of insertion of distal tip 608 (and the rest of device 600) into an incision and through tissue to a target site. In some embodiments, distal tip 608 may be squared off, pointed, or shaped in another manner.

Recess 606 may be concave, and may function as a cavity configured to receive an expandable device in a contracted or collapsed orientation. While a particular shape for recess 606 is shown, its shape may be different depending on the procedures for which insertion device 600 is intended to be used. For example, recess 606 may be sized and configured to accommodate a desired expandable device in a contracted/folded position. An expandable device may be coupled to recess 606 by, e.g., a biocompatible adhesive, or by any other suitable method.

In an exemplary method of use, a user may insert a distal tip (e.g., distal tip 608) of insertion device 600 into an incision. As insertion device 600 is pushed farther into the incision, an expandable device coupled to recess 606 may be advanced into the incision as well. A user may position the expandable device at a target site intended for tissue dissection, tissue expansion, or application of pressure. Once the expandable device is positioned at a target site, the expandable device may be deployed. Since the expandable device is coupled to recess 606, a user may be able to reposition the expandable device using insertion device 600 during the expansion process. This may be useful to resolve, e.g., issues of an expandable device snagging on body tissues or otherwise becoming caught or trapped in an undesirable location. A user may maneuver handle 602 to adjust the location of recess 606 and an expandable device coupled thereto.

In some examples, the expandable device is not disconnected from insertion device 600 during its deployment at the target site. For example, in cases in which the expandable device is a balloon, an inflation pump may be coupled to a proximal end of a flexible lumen, the distal end of the flexible lumen being in fluid communication with the balloon. The inflation pump may be coupled to the flexible lumen before or after introduction of insertion device 600 into an incision. During inflation of the balloon (or other expansion of an expandable device), insertion device 600 may remain inside the incision at the target site. After use of the expandable device is complete (e.g., after tissue is dissected, a tissue pocket or other cavity is enlarged, or pressure has been applied as desired, etc.), the expandable device is contracted/collapsed and is removed from the target site along with insertion device 600, by pulling insertion device 600 in a proximal direction by, e.g., handle 602.

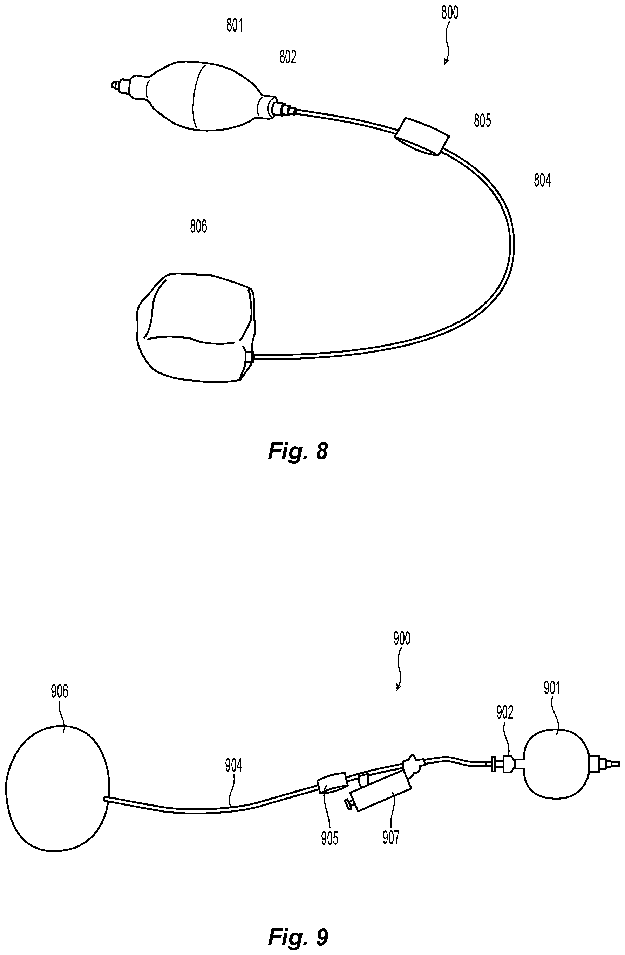

FIGS. 7, 8, and 9 illustrate various expandable devices and their components, according to further aspects of the present disclosure. In particular, FIG. 7 illustrates an exemplary expandable device 700 including a balloon 706, a flexible lumen 704, and an adapter 702 or other connection element to which a pump (not shown) may be connected. FIG. 8 illustrates an exemplary expandable device 800 including a balloon 806, a flexible lumen 804, an adapter 802 or other connection element to which a pump 801 is connected. A clip 805 is shown attached to lumen 804. FIG. 9 illustrates an exemplary expandable device 900 including a balloon 906, a flexible lumen 904, an adapter 902 or other connection element, and a pump 901 connected to adapter 902. A clip 905 and an adjustable clamp 907 are shown attached to lumen 904.

According to the present disclosure, expandable devices may include balloons, such as, e.g., balloons 706, 806, 906. The balloon of an expandable device may have a circular or other generally rounded shape when deflated, and a spherical, ovoid, or other shape when expanded. It will be understood, however, that any shaped balloon may be used for an expandable device. In some embodiments, the balloon may be shaped to create certain cavities within the dissected tissues depending on a desired procedure. A rounded shape may be particularly suitable, for example, for a target site in which a rounded implant is intended to be implanted. The balloon may have any suitable thickness and size. In some embodiments, the balloon may have a wall thickness of between about 0.1 mm and about 0.5 mm, such as about 0.1 mm, about 0.15 mm, about 0.2 mm, about 0.25 mm, about 0.3 mm, or about 0.35 mm. In some embodiments, the balloon may have an uninflated diameter of between about 10 cm and about 20 cm, such as between about 10 cm and about 15 cm, such as 10 cm, 12 cm, 14 cm, 16 cm, 18 cm, or 20 cm.

A flexible lumen (such as lumens 704, 804, 904) may extend from an outer wall of the balloon of an expandable device, and may be in fluid communication with an interior of the balloon. The lumen may be of any suitable length, such as, e.g., between about 20 cm and about 50 cm. For example, the lumen may have a length of between about 20 cm and about 40 cm, or between about 30 cm and about 40 cm, such as about 30 cm, about 32 cm, about 34 cm, about 36 cm, about 38 cm, or about 40 cm. The length of the flexible lumen may vary to ensure that the tube extends from the balloon positioned in the body cavity to the exterior of the patient.

The balloons and/or lumens disclosed herein may be made of, e.g., a flexible biocompatible polymer (e.g., silicone). In some embodiments, the balloon and lumen may be made from a single material, and in alternate embodiments, may be made from different materials.

Devices 800 and 900 are depicted as including manually-operated, unidirectional pumps 801, 901 attached to proximal end of lumens 804, 904 respectively, via adapters 802, 902, respectively. When a first side of each pump 801, 901 is coupled to lumen 804 or lumen 904, utilizing the pumps may inflate the respective balloons 806, 906 connected to the pumps via, e.g., lumens 804, 904. In particular, when the first side of each pump 801, 901 is coupled to respective lumens 804, 904, actuating pumps 801, 901 may inflate balloons 806, 906. Attaching the opposite end of pumps 801, 901 to lumens 804, 904 may deflate balloons 806, 906 when pumps 801, 901 are actuated.

Pumps 801, 901 may expand balloons 806, 906 using any other suitable method or mechanism, such as pressurized air or pressurized fluid, and may be electrically operated, mechanically operated, or a combination thereof. Clips 805, 905 and/or adjustable clamp 907 may be used on lumens 804, 904 to prevent the balloons 806, 906 from deflating once inflated.

Optionally, a string may be provided in a lumen (e.g., lumens 804, 904), a first end of which is attached at an interior equator point of a balloon (e.g., balloons 806, 906), and a second end of which extends through the lumen (e.g., lumens 804, 904) and out the proximal end. As the balloon is expanded, the string is pulled into the lumen, thereby allowing a user to measure expansion of the balloon by using markings on the opposite end extending out the proximal end of the tube. As the balloon is inflated, the balloon expands and the string is pulled into the tubing, thereby allowing the physician to measure the expansion of the balloon. In some embodiments, markings on the lumen may be provided to aid in measuring movement of the string.

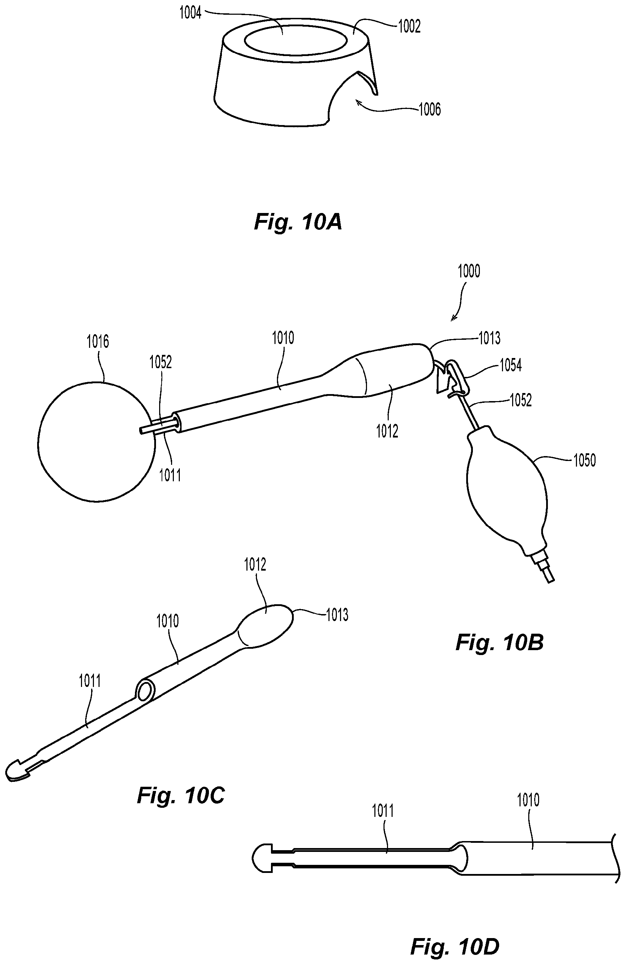

FIGS. 10A-10D illustrate views of another exemplary insertion device, its components, and accessories, according to aspects of the present disclosure and in correspondence with the present claimed invention. FIG. 10A depicts a tapered cup 1002, the cup including an interior 1004 and a side opening 1006. FIG. 10B depicts an insertion device assembly 1000, including an insertion device 1010 having a handle, e.g., bulb-shaped grip 1012 at or proximate the proximal end 1013 of the device 1010, and a projection 1011 extending distally. A balloon 1016 is coupled to projection 1011 and is in fluid communication with flexible lumen 1052, which passes through a cavity defined by a tube of insertion device 1010 and out of proximal end 1013. At least a portion of the balloon 1016 optionally may be disposed within the cavity (e.g., within the tube) while a portion of the balloon is coupled to the projection 1011. A proximal end of lumen 1052 is connected to a pump 1050. A clip 1054 is disposed on lumen 1052. Figures 10C and 10D depict additional views of insertion device 1010, and in particular distal projection 1011, which may include a tapered distal tip.

Insertion device assembly 1000 may be utilized as described with respect to any other examples of insertion devices disclosed herein. Materials, sizes, shapes, characteristics, measurements of parts of insertion device assembly 1000 (e.g., insertion device 1010, balloon 1016, lumen 1052, pump 1050, and clip 1054) may likewise correspond to materials, sizes, shapes, characteristics, and measurements of any other like components of insertion devices disclosed herein. For example, insertion device 1010 may include depth markers or measurement markers on its surface to assist a user in determining an extent to which insertion device has been extended into an incision. As with other exemplary insertion devices disclosed herein, insertion device 1010 may have a longitudinal length (extending along a proximal-distal axis) ranging from about 15 cm to about 35 cm, such as from about 18 cm to about 32 cm, from about 18 cm to about 30 cm, from about 18 cm to about 28 cm, from about 18 cm to about 25 cm, from about 19 cm to about 23 cm, or from about 19 cm to about 21 cm, such as about 16 cm, about 18 cm, about 20 cm, about 22 cm, about 24 cm, about 26 cm, about 28 cm, about 30 cm, about 32 cm, or about 34 cm. As is the case with other devices disclosed herein, the cross-sectional dimensions (perpendicular to a longitudinal length measured along a proximal-distal axis) of insertion device 1010 may be small (e.g., less than about 3 cm, less than about 2 cm, or less than about 1 cm across).

The tapered or anchor-shaped tip of insertion device 1010 may assist in insertion of device 1010 through an incision. Additionally and/or alternately, a narrowed portion of the tip on projection 1011 may be used to couple projection 1011 to an expandable device, such as balloon 1016. Projection 1011 may be disposed inside, or may be coupled, adhered, or affixed to an exterior of balloon 1016.

Cup 1002 may be configured for use with insertion device assembly 1000. An internal circumference, diameter, or volume of cup 1002 may correspond approximately to a corresponding circumference, diameter, or volume of an implant to be introduced at a target site. Cup 1002 may be placed over the exterior of the target site prior to deployment of the expandable device (e.g., balloon 1016) within the target site. As balloon 1016 is expanded inside the target site, cup 1002 may limit expansion by externally restricting expansion of the patient's tissue at the target site. Once balloon 1016 is expanded to the extent allowed by cup 1002, clip 1054 may be engaged to pinch lumen 1052 and restrict fluid flow out of balloon 1016, maintaining a size and shape of balloon 1016 corresponding to a desired size and shape as delineated by cup 1002. In this manner, insertion device assembly 1000, in combination with cup 1002, may be used to insert and expand an expandable device to an extent necessary to conform patient tissue to a desired shape and size. Once this is accomplished, an implant may be selected for implantation into the space created by the expandable device of insertion device assembly 1000.

As will be understood by one of ordinary skill in the art, cup 1002 may have any size or shape suitable to delineate a desired size or shape of patient tissue. In some embodiments, cup 1002, and the size and shape of interior 1004, may be customized for a particular patient. Cup 1002 may include one or more openings, such as side opening 1006. Side opening 1006 may allow for cup 1002 to be placed over a target site without covering, e.g., the incision for insertion of insertion device 1010.

Any device described herein optionally may employ a biocompatible lubricant and/or a lubricious coating, in any suitable form, to aid in reducing friction caused by movement of the device against other components and/or against tissue. Additionally, any device disclosed herein may include markings, such as depth markings, incremental measurements, and the like to aid a user (e.g., a physician) in visually checking and adjusting a position of the device in an incision, and/or deployment of an expandable device at a target site. Furthermore, any device disclosed herein may include light-emitting devices, radio-opaque markings, or other indicators positioned at, e.g., a distal end portion of the device to aid in positioning the device at a target site (e.g., between tissue layers or planes to be dissected). In addition, any feature of an exemplary device described herein may be combined with any other features and/or described devices. For example, cup 1002 may be used with insertion devices 100, 200, 320, 500, and/or 600.

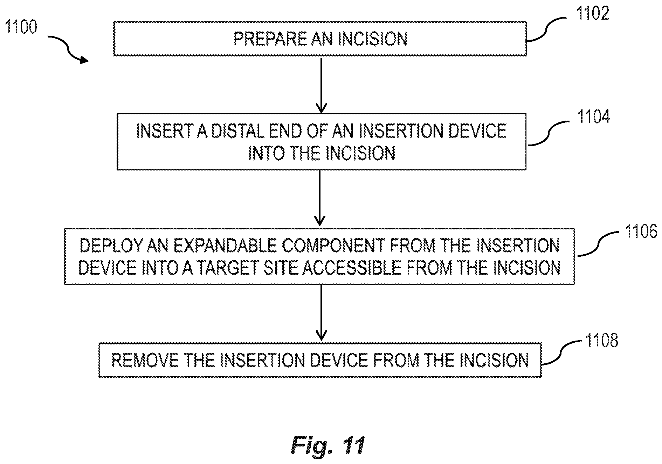

While aspects of the use of various insertion devices have already been described herein, FIG. 11 depicts, in flow chart form, an exemplary general method 1100 for inserting an expandable device using an insertion device according to aspects of the present disclosure. Method 1100, and variations thereof, may be applicable to any insertion device described or encompassed by this disclosure, as well as other insertion devices. It will be contemplated by those of ordinary skill in the art that FIG. 11 depicts merely an exemplary method, of which many variations are possible. In some embodiments, one or more steps of FIG. 11 may be added, removed, duplicated, or performed out of order. The steps of method 1100, and variations thereon, may be performed by one or more users, such as medical professionals, technicians, assistants, etc.

According to step 1102 of method 1100, an incision may be prepared. This may be done by, e.g., a physician, such as an access surgeon or other surgeon performing a medical procedure. In some embodiments, as has been described elsewhere herein, the incision may be a minimally invasive incision, configured to be just large enough to allow for an insertion instrument to be inserted. For example, an incision according to the present disclosure may be about 5 cm or less in length, about 4 cm or less, about 3 cm or less, about 2 cm or less, or about 1 cm or less. For example, in some embodiments, the incision may be between about 1.5 and about 2.5 cm, such as 1.5 cm, 1.7 cm, 1.9 cm, 2.1 cm, 2.3 cm, or 2.5 cm.

According to step 1104, a distal end of an insertion device may be inserted into the incision. As has been described elsewhere herein, a distal end of an insertion device according to the present disclosure may be tapered and/or rounded to allow for ease of insertion. The insertion device may then be advanced distally through the incision until the distal end portion reaches a desired position (e.g., at or near a position where an expandable device is intended to be deployed, such as a target site). According to step 1106, an expandable component (e.g., the expandable device) may be deployed from the insertion device (e.g., a cavity in the insertion device) into the target site accessible from the incision. According to step 1108, the insertion device may be removed from the incision. As has been described elsewhere herein, the insertion device may be removed from the incision before the expandable component is removed from the target site. In additional or alternative embodiments, the insertion device is not removed until the expandable component is contracted (e.g., deflated, compressed, etc.) and retracted back into the insertion device (e.g., into a cavity in the insertion device). Thus, in such examples, the insertion device may be used to withdraw the expandable component.

While principles of the present disclosure are described herein with reference to illustrative aspects for particular applications, it should be understood that the disclosure is not limited thereto. Those having ordinary skill in the art and access to the teachings provided herein will recognize additional modifications, applications, aspects, and substitution of equivalents all fall within the scope of the aspects described herein. Accordingly, the present disclosure is not to be considered as limited by the foregoing description. The invention is defined by the following claims.

## Claims

1. An insertion device (1000), comprising:
a base (1010) having a longitudinal axis and defining a cavity, wherein the base includes a plurality of measurement markings;
an expandable balloon (1016) disposed within the cavity in a collapsed configuration; and
a flexible lumen (1052) extending proximally from the balloon to a pump, the flexible lumen fluidly connecting the balloon to the pump;
wherein the base includes a projection (1011) extending distally, parallel to the longitudinal axis,
wherein at least a portion of the balloon is coupled to the projection.

2. The device of claim 1, wherein the balloon is affixed to the projection with an adhesive.

3. The device of claim 1 or 2, wherein a longitudinal length of the projection is at least half a longitudinal length of a tube that defines the cavity.

4. The device of any of the preceding claims, wherein the flexible lumen passes through the cavity defined by the tube.

5. The device of any of the preceding claims, wherein a distal end (154, 554) of the base includes a tapered atraumatic tip.

6. The device of any of the preceding claims, wherein a proximal portion of the base includes a handle (1012).

7. The device of any of the preceding claims, wherein a diameter of the balloon in the collapsed configuration is between about 10 mm and about 15 mm.

8. The device of any of the preceding claims, wherein the balloon is in fluid communication with a supply of air.

9. The device of any of the preceding claims, wherein a cross-sectional dimension of the base is less than about 3 cm, such as from about 0.5 cm to 3 cm.

10. The device of any of the preceding claims, wherein the device has a length ranging from about 15 cm to about 35 cm.

11. The device of any of the preceding claims, further comprising a clip (1054) disposed on the flexible lumen (1052) to prevent the balloon from deflating once inflated.

12. The device of any of the preceding claims, wherein the base includes a bulb-shaped grip (1012) at or proximate a proximal end (1013) of the base.

13. A kit, comprising:
the device of any of the preceding claims; and
a cup (1002) defining an interior volume, the cup configured to be placed external to a target site and to limit expansion of the balloon inside the target site.

14. The kit of claim 13, wherein the cup includes a side opening.

## Patentansprüche

1. Einsatzvorrichtung (1000), umfassend:
einen Boden (1010), der eine Längsachse aufweist und einen Hohlraum definiert, wobei der Boden eine Vielzahl von Messmarkierungen beinhaltet;
einen expandierbaren Ballon (1016), der in einer kollabierten Konfiguration innerhalb des Hohlraums angeordnet ist; und
ein flexibles Lumen (1052), das sich proximal von dem Ballon zu einer Pumpe erstreckt, wobei das flexible Lumen den Ballon fluidisch mit der Pumpe verbindet;
wobei der Boden einen Vorsprung (1011) beinhaltet, der sich distal, parallel zur Längsachse erstreckt, wobei zumindest ein Abschnitt des Ballons mit dem Vorsprung gekoppelt ist.

2. Vorrichtung nach Anspruch 1, wobei der Ballon mit einem Klebstoff an dem Vorsprung befestigt ist.

3. Vorrichtung nach Anspruch 1 oder 2, wobei die Länge des Vorsprungs in Längsrichtung mindestens die Hälfte der Länge eines den Hohlraum definierenden Schlauchs in Längsrichtung beträgt.

4. Vorrichtung nach einem der vorhergehenden Ansprüche, wobei das flexible Lumen durch den durch den Schlauch definierten Hohlraum hindurchgeht.

5. Vorrichtung nach einem der vorhergehenden Ansprüche, wobei ein distales Ende (154, 554) des Bodens eine sich verjüngende atraumatische Spitze beinhaltet.

6. Vorrichtung nach einem der vorhergehenden Ansprüche, wobei ein proximaler Abschnitt des Bodens einen Griff (1012) beinhaltet.

7. Vorrichtung nach einem der vorhergehenden Ansprüche, wobei ein Durchmesser des Ballons in der kollabierten Konfiguration zwischen etwa 10 mm und etwa 15 mm beträgt.

8. Vorrichtung nach einem der vorhergehenden Ansprüche, wobei der Ballon in Fluidverbindung mit einer Luftzufuhr steht.

9. Vorrichtung nach einem der vorhergehenden Ansprüche, wobei eine Querschnittsabmessung des Bodens weniger als etwa 3 cm, wie etwa 0,5 cm bis 3 cm, beträgt.

10. Vorrichtung nach einem der vorhergehenden Ansprüche, wobei die Vorrichtung eine Länge im Bereich von etwa 15 cm bis etwa 35 cm hat.

11. Vorrichtung nach einem der vorhergehenden Ansprüche, ferner umfassend eine Klammer (1054), die an dem flexiblen Lumen (1052) angeordnet ist, um zu verhindern, dass sich der Ballon nach dem Aufblasen entleert.

12. Vorrichtung nach einem der vorhergehenden Ansprüche, wobei der Boden einen knollenförmigen Griff (1012) an oder in der Nähe eines proximalen Endes (1013) des Bodens beinhaltet.

13. Kit, umfassend:
die Vorrichtung nach einem der vorhergehenden Ansprüche; und
einen Becher (1002), der ein Innenvolumen definiert, wobei der Becher konfiguriert ist, um außerhalb einer Zielstelle platziert zu werden und um die Expansion des Ballons innerhalb der Zielstelle zu begrenzen.

14. Kit nach Anspruch 13, wobei der Becher eine Seitenöffnung beinhaltet.

## Revendications

1. Dispositif d'insertion (1000), comprenant :
une base (1010) ayant un axe longitudinal et définissant une cavité, dans lequel la base comprend une pluralité de marquages de mesure ;
un ballonnet expansible (1016) disposé à l'intérieur de la cavité dans une configuration repliée ; et
une lumière souple (1052) s'étendant proximalement du ballonnet à une pompe, la lumière souple reliant fluidiquement le ballonnet à la pompe ;
dans lequel la base comprend une saillie (1011) s'étendant distalement, parallèle à l'axe longitudinal, dans lequel au moins une partie du ballonnet est couplée à la saillie.

2. Dispositif selon la revendication 1, dans lequel le ballonnet est fixé à la saillie par un adhésif.

3. Dispositif selon la revendication 1 ou 2, dans lequel la longueur longitudinale de la saillie est au moins égale à la moitié de la longueur longitudinale d'un tube qui définit la cavité.

4. Dispositif selon l'une quelconque des revendications précédentes, dans lequel la lumière souple traverse la cavité définie par le tube.

5. Dispositif selon l'une quelconque des revendications précédentes, dans lequel l'extrémité distale (154, 554) de la base comprend une pointe atraumatique effilée.

6. Dispositif selon l'une quelconque des revendications précédentes, dans lequel la partie proximale de la base comprend une poignée (1012).

7. Dispositif selon l'une quelconque des revendications précédentes, dans lequel le diamètre du ballonnet en configuration repliée est compris entre environ 10 mm et environ 15 mm.

8. Dispositif selon l'une quelconque des revendications précédentes, dans lequel le ballonnet est en communication fluidique avec une alimentation en air.

9. Dispositif selon l'une quelconque des revendications précédentes, dans lequel la dimension de la section de la base est inférieure à environ 3 cm, telle que comprise entre environ 0,5 cm et 3 cm.

10. Dispositif selon l'une quelconque des revendications précédentes, dans lequel le dispositif a une longueur comprise entre environ 15 cm et environ 35 cm.

11. Dispositif selon l'une quelconque des revendications précédentes, comprenant également une pince (1054) disposée sur la lumière souple (1052) pour empêcher le ballonnet de se dégonfler une fois gonflé.

12. Dispositif selon l'une quelconque des revendications précédentes, dans lequel la base comprend une poignée en forme de bulbe (1012) au niveau ou à proximité d'une extrémité proximale (1013) de la base.

13. Kit, comprenant :
le dispositif selon l'une quelconque des revendications précédentes ; et
une coupelle (1002) définissant un volume intérieur, la coupelle étant configurée pour être placée à l'extérieur d'un site cible et pour limiter l'expansion du ballonnet à l'intérieur du site cible.

14. Kit selon la revendication 13, dans lequel la coupelle comprend une ouverture latérale.
